# EUROPEAN PATENT APPLICATION

(11) **EP 4 663 749 A1**
(43) Date of publication of application: **17.12.2025**
(21) Application number: 24780966.8
(22) Date of filing: 04.01.2024
(51) Int. Cl.: C12N 5/0775, C12N 5/04

(54) **METHOD FOR PROMOTING SECRETION OF EXOSOMES FROM ANIMAL CELLS BY USING ROSE-DERIVED EXOSOMES**

(30) Priority: 30.03.2023 KR 20230042359; 03.01.2024 KR 20240000846
(71) Applicant: Exocobio Inc., Cheongju-si, Chungcheongbuk-do 28161 (KR)
(72) Inventor: CHO, Byong Seung, Anyang-si, Gyeonggi-do 14102 (KR)
(74) Representative: Isarpatent
(86) International application number: PCT/KR2024/000181
(87) International publication number: WO 2024/204971

(57) **Abstract**

The present invention provides a method for promoting exosome secretion from animal cells, comprising treating the animal cells with rose-derived exosomes. According to the present invention, the secretion of exosomes from animal cells can be promoted by treating the animal cells with rose-derived exosomes.

## Description

### TECHNICAL FIELD

The present invention relates to a method for promoting exosome secretion from animal cells using rose-derived exosomes.

Moreover, the present invention relates to a medium for promoting exosome secretion from animal cells, comprising rose-derived exosomes.

### BACKGROUND ART

Recently, there have been reports that cell secretomes contain various bioactive molecules that regulate cellular behaviors. In particular, cell secretomes contain 'exosome' or 'extracellular vesicle' that has intercellular signaling functions, and thus studies on the components and functions thereof have been actively conducted.

Cells shed various membraneous vesicles to their extracellular environment, and these released vesicles are usually called extracellular vesicles (EVs). The EV is also called cell membrane-derived vesicle, ectosome, shedding vesicle, microparticle, exosome, etc., and is also used discriminately from exosome in some cases. Depending on the isolation environment, conditions, and methods, extracellular vesicles may have the same meaning as exosomes, and may also be meant to include nanovesicles that have the same or similar size as exosomes, but have a composition which differs from that of exosomes.

Exosome is a vesicle of tens to hundreds of nanometers in size, which comprises a phospholipid bilayer membrane having the same structure as that of the cell membrane. This exosome contains proteins, nucleic acids (mRNA, miRNA, etc.) and the like which are called exosome cargo. It is known that exosome cargo includes a wide range of signaling factors, and these signaling factors are specific for cell types and regulated differently depending on secretory cells' environment. It is known that exosome is an intercellular signaling mediator secreted by cells, and various cellular signals transmitted through it regulate cellular behaviors, including the activation, growth, migration, differentiation, dedifferentiation, apoptosis, and necrosis of target cells. Exosome contains specific genetic materials and bioactive factors depending on the nature and state of cells from which the exosome was derived. Exosome derived from proliferating stem cells regulates cell behaviors such as cell migration, proliferation and differentiation, and recapitulates the characteristics of stem cells involved in tissue regeneration (Nature Review Immunology 2002 (2) 569-579).

That is, exosomes called "avatars" of cells contain bioactive factors such as growth factors, similar to cells, and serve as carriers that transmit bioactive factors between cells, that is, serve to mediate cell-to-cell communication. Exosomes are known to be released not only from animal cells such as stem cells, immune cells, fibroblasts, skin cells, and cancer cells, but also from cells of various organisms such as plants, bacteria, fungi, and algae. For example, exosomes can be isolated from conditioned media of plant cells, conditioned media of plant callus, conditioned media of plant stem cells, plant juice, or a biological solution of a plant equivalent thereto, as well as conditioned media of stem cells, immune cells, fibroblasts, skin cells, cancer cells or the like.

Meanwhile, roses are cultivated in a wide range of areas such as cold, subarctic, temperate, and subtropical zones of the northern hemisphere, and their extracts are used in perfumes and cosmetics. Rose stem cell-derived exosomes are known to exhibit efficacy in skin regeneration, skin elasticity improvement, and wrinkle reduction (Korean Patent Publication No. 10-2058444), anti-inflammatory efficacy (Korean Patent Publication No. 10-2341932), and skin whitening efficacy (Korean Patent Publication No. 10-2261434). However, further detailed research on the features and functions of rose-derived exosomes is needed.

The present inventor has conducted intensive research on rose-derived exosomes and, as a result, have completed the present invention by confirming that treating animal cells with rose-derived exosomes during culture of the animal cells promotes exosome secretion from the animal cells.

Meanwhile, it is to be understood that the matters described as the background art are intended merely to aid in the understanding of the background of the present invention and are not admitted as prior art against the present invention.

### SUMMARY OF INVENTION

An object of the present invention is to provide a method for promoting exosome secretion from animal cells using rose-derived exosomes.

Another object of the present invention is to provide a medium for promoting exosome secretion from animal cells, comprising rose-derived exosomes.

However, the objects of the present invention as described above are illustrative and the scope of the present invention is not limited thereby. In addition, other objects and advantages of the present invention will be more apparent from the following description, the appended claims and the accompanying drawings.

### DETAILED DESCRIPTION OF INVENTION

As used herein, the term "rose (Rosa spp.)" refers to plants belonging to the genus Rosa, in the family Rosaceae, the order Rosales and the class Dicotyledoneae, and includes all of wild species and cultivated garden species.

As used herein, the term "exosomes" refers to vesicles of tens to hundreds of nanometers in size (preferably, about 30 to 200 nm), which comprise a phospholipid bilayer membrane having the same structure as that of the cell membrane (however, the particle size of exosomes is variable depending on the type of cell from which the exosomes are isolated, an isolation method and a measurement method) (Vasiliy S. Chernyshev et al., "Size and shape characterization of hydrated and desiccated exosomes", Anal Bioanal Chem, (2015) DOI 10.1007/s00216-015-8535-3). These exosomes contain proteins, nucleic acids (mRNA, miRNA, etc.) and the like which are called exosome cargo. It is known that exosome cargo includes a wide range of signaling factors, and these signaling factors are specific for cell types and regulated differently depending on secretory cells' environment. It is known that exosomes are intercellular signaling mediators secreted by cells, and various cellular signals transmitted through them regulate cellular behaviors, including the activation, growth, migration, differentiation, dedifferentiation, apoptosis, and necrosis of target cells.

In the case of plants such as roses, "exosomes" refer to nano-sized vesicles secreted or released from plant cells into extracellular spaces and having a membrane structure, also called extracellular vesicles, exosome-like vesicles, or exosome-like particles.

Furthermore, in the case of animal cells, "exosomes" include not only extracellular vesicles secreted from animal cells and released into the extracellular space, having a phospholipid bilayer membrane structure and specific markers, but also vesicles of nano-sized vesicle structure having a similar composition to exosomes (e.g., exosome-like vesicles or exosome-like particles).

As used herein, the term "exosome" used in relation to exosome secretion promotion is used in a sense encompassing extracellular vesicles.

As used herein, the term "rose-derived exosomes" means to include all exosomes isolated from, for example, a conditioned medium of rose plant cells, a conditioned medium of rose callus, a conditioned medium of rose plant stem cells, rose juice, or a biological solution of rose equivalent thereto, or derived (e.g., secreted or released) from rose plant cells or rose plant stem cells.

In the present invention, the type of animal cells from which exosomes are secreted is not limited, but as an example not limiting the scope of the present invention, they may be stem cells, immune cells, or skin cells. The stem cells may be embryonic stem cells, induced pluripotent stem cells (iPSC), adult stem cells, embryonic stem cell-derived mesenchymal stem cells, or induced pluripotent stem cell-derived mesenchymal stem cells. The immune cells may be T cells, B cells, NK cells, cytotoxic T cells, dendritic cells, or macrophages. The skin cells may be keratinocytes or dermal fibroblasts.

As an example not limiting the scope of the present invention, the adult stem cells may be at least one type of adult stem cells selected from the group consisting of mesenchymal stem cells, human-derived mesenchymal stromal cells, human-derived mesenchymal stem cells, and multipotent stem cells. The mesenchymal stem cells may be mesenchymal stem cells derived from at least one tissue selected from the group consisting of umbilical cord, umbilical cord blood, bone marrow, adipose tissue, muscle, nerve, skin, amniotic membrane, Wharton's jelly, and placenta. Preferably, the adult stem cells may be mesenchymal stem cells, for example, adipose-, bone marrow-, umbilical cord- or umbilical cord blood-derived stem cells, more preferably adipose-derived stem cells, and even more preferably human adipose-derived stem cells. The type of stem cells, immune cells, or skin cells is not limited as long as they do not pose a risk of pathogen infection and do not cause immune rejection, but may preferably be human-derived stem cells, human-derived immune cells, or human-derived skin cells.

However, it is of course possible to use various animal cells that are being used in the art or may be used in the future, as long as they do not cause adverse effects on the human body. For example, it is also possible to use HEK293 cells or HEK293T cells. Thus, it is to be understood that the human adipose-derived stem cell used in the Examples described later is an example of animal cells that may be used in the present invention, and the present invention is not limited thereto.

The present invention provides a method for promoting exosome secretion from animal cells, comprising treating the animal cells with rose-derived exosomes.

In the method for promoting exosome secretion from animal cells according to one embodiment of the present invention, treating the animal cells with rose-derived exosomes may comprise adding the rose-derived exosomes to a culture medium of the animal cells, and culturing the animal cells in the culture medium.

In the method for promoting exosome secretion from animal cells according to one embodiment of the present invention, the exosomes secreted from the animal cells may be quantified by the exosome content (tetraspanin content, e.g., CD81 content) per animal cell (or animal cell conditioned medium), or the number of exosome particles per animal cell (or animal cell conditioned medium).

The present invention provides a medium for promoting exosome secretion from animal cells, comprising rose-derived exosomes.

### ADVANTAGEOUS EFFECTS

According to the present invention, treating animal cells with rose-derived exosomes can promote exosome secretion from the animal cells.

It should be understood that the scope of the present invention is not limited to the aforementioned effects.

### BRIEF DESCRIPTION OF DRAWINGS

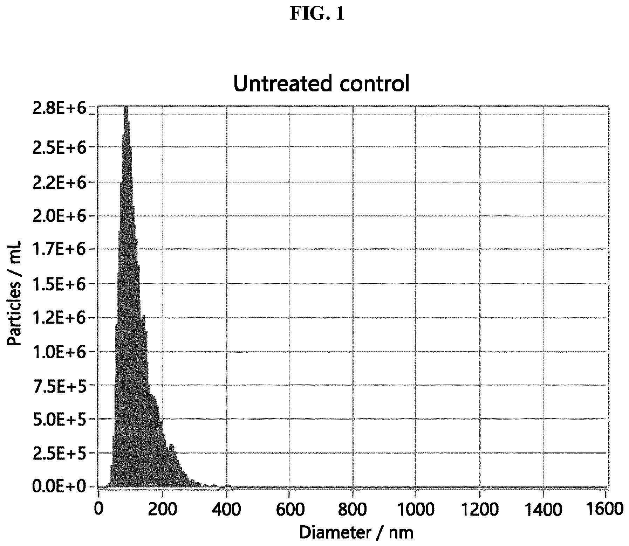

FIG. 1 is a graph showing the particle size distribution and particle number obtained by performing nanoparticle tracking analysis (NTA) of a culture supernatant obtained after culturing human adipose-derived stem cells in a medium not containing rose-derived exosomes (hereinafter referred to as "untreated control" or "control").

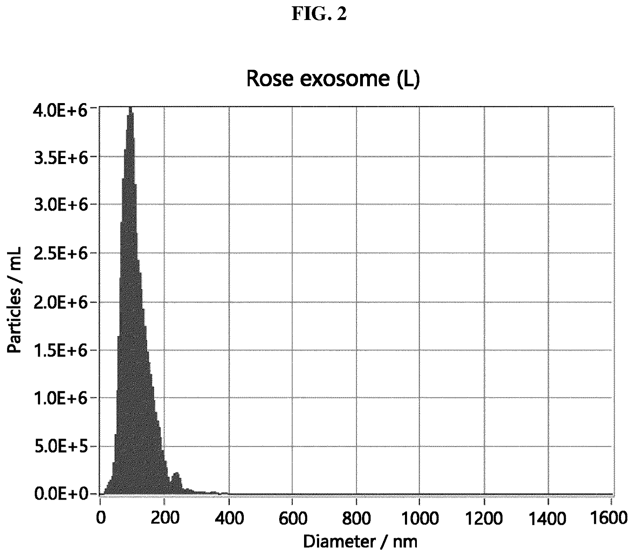

FIG. 2 is a graph showing the particle size distribution and particle number obtained by performing nanoparticle tracking analysis (NTA) of a culture supernatant obtained after culturing human adipose-derived stem cells in a medium supplemented with a low concentration of rose-derived exosomes.

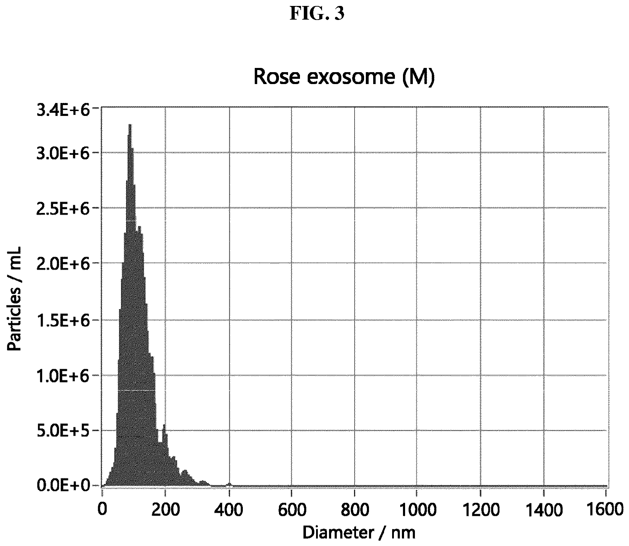

FIG. 3 is a graph showing the particle size distribution and particle number obtained by performing nanoparticle tracking analysis (NTA) of a culture supernatant obtained after culturing human adipose-derived stem cells in a medium supplemented with a moderate concentration of rose-derived exosomes.

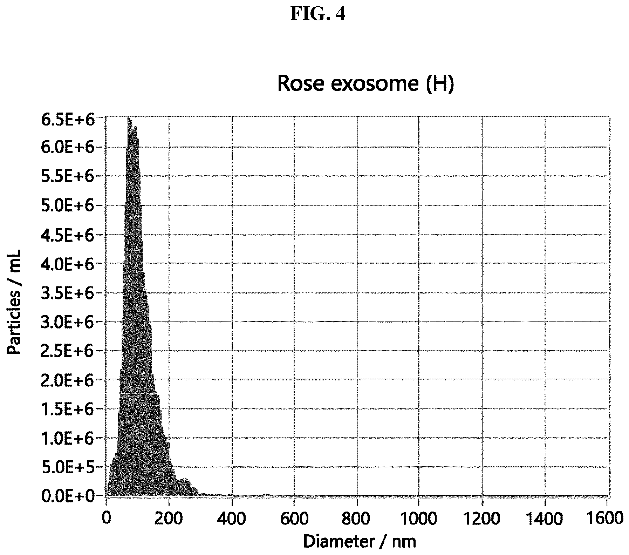

FIG. 4 is a graph showing the particle size distribution and particle number obtained by performing nanoparticle tracking analysis (NTA) of a culture supernatant obtained after culturing human adipose-derived stem cells in a medium supplemented with a high concentration of rose-derived exosomes.

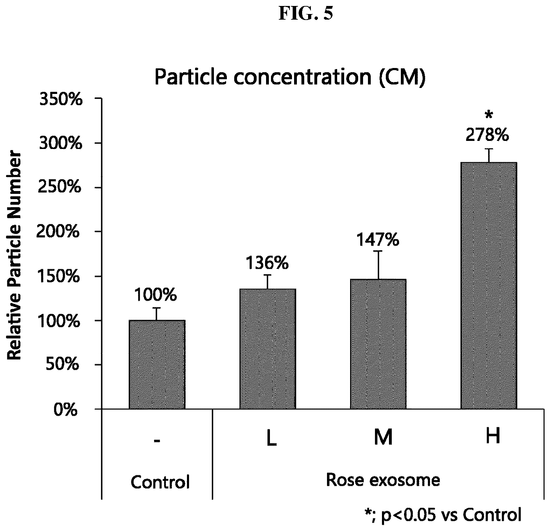

FIG. 5 is a comparative graph showing that the number of particles per mL of conditioned medium (i.e., the amount of exosome secretion) remarkably increased compared to the untreated control when human adipose-derived stem cells were cultured in a medium containing rose-derived exosomes according to one embodiment of the present invention.

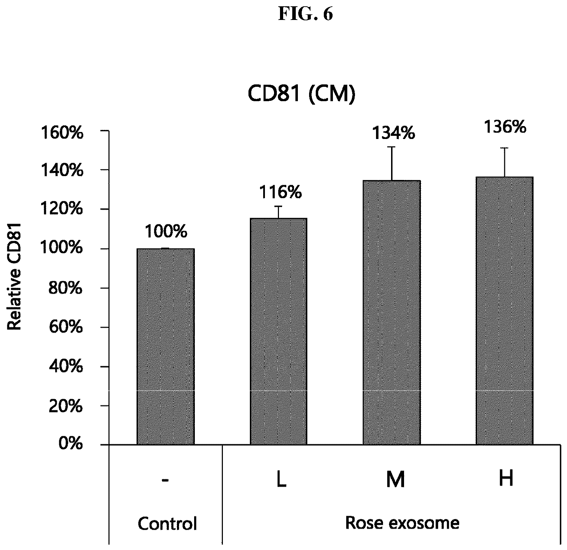

FIG. 6 is a comparative graph showing that the exosome content (content of CD81, which is an exosome marker) per mL of conditioned medium increased compared to the untreated control when human adipose-derived stem cells were cultured in a medium containing rose-derived exosomes according to one embodiment of the present invention.

### EXAMPLES

Hereinafter, the present invention will be described in more detail with reference to the following examples. However, the following examples are only to illustrate the present invention and are not intended to limit or restrict the scope of the present invention. Those that can be easily inferred by those skilled in the art from the detailed description and examples of the present invention are interpreted as falling within the scope of the present invention. References referred to in the present invention are incorporated herein by reference.

Throughout the present specification, it is to be understood that, when any part is referred to as "comprising" any component, it does not exclude other components, but may further include other components, unless otherwise specified.

### Example 1: Preparation of Rose Callus and Rose-Derived Exosomes

According to plant callus preparation and culture methods known in the art, calluses were induced from rose petals, leaves, stems, roots and/or embryos, and cells of the induced rose callus were cultured. Then, a callus having good growth status was selected and cultured in large amounts to prepare conditioned medium of rose callus. The conditioned medium of rose callus was filtered through a 0.22 µm filter to remove impurities such as cell debris, waste products, and large particles. Rose-derived exosomes were isolated from the filtered conditioned medium using a tangential flow filtration (TFF) method.

### Example 2: Cell Culture

Human adipose-derived stem cells were suspended in DMEM culture medium containing 10% fetal bovine serum (FBS), 100 Units/mL penicillin and 100 µg/mL streptomycin. They were then inoculated into a flask and cultured in an incubator at 37°C under 5% CO₂. For the low, moderate, and high concentration treatment groups of rose-derived exosomes, rose-derived exosomes were added to the culture medium at final concentrations of 2.5×10⁸ particles/mL (indicated as "L" in FIGS. 2, 5 and 6), 8.0×10⁸ particles/mL (indicated as "M" in FIGS. 3, 5 and 6), and 2.5×10⁹ particles/mL (indicated as "H" in FIGS. 4, 5 and 6), respectively.

When the cells reached a confluency of 80% or more, the cells were washed with phosphate-buffered saline (PBS; purchased from Thermo Scientific), and then the culture medium was replaced with DMEM medium containing 100 Units/mL penicillin and 100 µg/mL streptomycin.

Thereafter, human adipose-derived stem cells were cultured for 24 to 72 hours in the untreated control group (indicated as "Control" in FIGS. 5 and 6) and in the low-concentration, moderate-concentration, and high-concentration rose-derived exosome treatment groups, respectively. The supernatant of culture medium from each group was collected. After the supernatant collection was completed, the cell numbers of the untreated control group and the low-concentration, moderate-concentration, and high-concentration rose-derived exosome treatment groups were measured using a cell counter, respectively.

### Example 3: Analysis of Exosome Particle Size and Distribution, and Evaluation of Exosome Secretion Amount

For each of the supernatants collected from the untreated control group and the low-concentration, moderate-concentration, and high-concentration rose-derived exosome treatment groups in Example 2, the particle size and concentration were measured by performing nanoparticle tracking analysis (NTA) using ZetaView (purchased from Particle Metrix). The measured NTA results are shown in FIGS. 1 to 4. It was confirmed that the number of exosome particles in the collected supernatant increased by 36% in the low-concentration rose-derived exosome treatment group, 47% in the moderate-concentration rose-derived exosome treatment group, and 178% in the high-concentration rose-derived exosome treatment group, respectively, compared to the untreated control (FIG. 5).

Therefore, it was confirmed that when human adipose-derived stem cells were cultured in a medium containing rose-derived exosomes according to one embodiment of the present invention, i.e., when human adipose-derived stem cells were treated with rose-derived exosomes, the number of exosome particles (i.e., the amount of exosome secretion) remarkably increased compared to the untreated control.

### Example 4: Evaluation of Exosome Secretion Amount Using Exosome-Specific Marker

Since CD81 is a representative positive marker of exosomes, the CD81 content and the exosome content are linearly proportional to each other. An increase in CD81 content indicates a proportional increase in exosome content. Hereinafter, according to this principle, the exosome content (i.e., the amount of exosome secretion) was analyzed for each of the supernatants from the untreated control group and the low-concentration, moderate-concentration, and high-concentration rose-derived exosome treatment groups collected in Example 2. For this purpose, Exosome-Human CD81 Flow Detection Reagent was used. Each supernatant was mixed with Exosome-Human CD81 Flow Detection Reagent overnight, and then each mixture was reacted with PE Mouse anti-Human CD81 for 1 hour. After the reaction, the PE mean fluorescence intensity (MFI) of the samples was measured using flow cytometry.

Meanwhile, linear regression analysis was performed to calculate the CD81 content using the PE mean fluorescence intensity. That is, linear regression analysis was performed using the concentrations of serially diluted CD81 protein and the corresponding MFI values. The exosome content (CD81 content) in each supernatant was determined using the standard quantitative analysis graph generated from the linear regression analysis.

As shown in FIG. 6, a comparison of CD81 content confirmed that when human adipose-derived stem cells were cultured in a medium containing rose-derived exosomes (i.e., treated with rose-derived exosomes), the exosome content (CD81 content) in the supernatant increased compared to the untreated control.

Therefore, it was re-confirmed that when human adipose-derived stem cells were cultured in a medium containing rose-derived exosomes according to one embodiment of the present invention, i.e., when human adipose-derived stem cells were treated with rose-derived exosomes, the amount of exosome secretion increased.

Although the present invention has been described with reference to the embodiments, the scope of the present invention is not limited to these embodiments. Any person skilled in the art will appreciate that various modifications and changes are possible without departing from the spirit and scope of the present invention and these modifications and changes also fall within the scope of the present invention.

## Claims

1. A method for promoting exosome secretion from animal cells, comprising treating the animal cells with rose-derived exosomes.

2. The method of claim 1, wherein treating the animal cells with rose-derived exosomes comprises adding the rose-derived exosomes to a culture medium of the animal cells, and culturing the animal cells in the culture medium.

3. The method of claim 1 or 2, wherein the rose-derived exosomes are isolated from a conditioned medium of rose plant cells, a conditioned medium of rose callus, a conditioned medium of rose plant stem cells, rose juice, or a biological solution of rose equivalent thereto, or derived from rose plant cells or rose plant stem cells.

4. A medium for promoting exosome secretion from animal cells, comprising rose-derived exosomes.

5. The medium of claim 4, wherein the rose-derived exosomes are isolated from a conditioned medium of rose plant cells, a conditioned medium of rose callus, a conditioned medium of rose plant stem cells, rose juice, or a biological solution of rose equivalent thereto, or derived from rose plant cells or rose plant stem cells.
